(19) Europäisches Patentamt
European Patent Office
Office européen des brevets

(11) Publication number : **0 370 740 B1**

(12) # EUROPEAN PATENT SPECIFICATION

(45) Date of publication of patent specification : **29.07.92 Bulletin 92/31**

(21) Application number : **89312025.3**

(22) Date of filing : **20.11.89**

(51) Int. Cl.$^5$ : **C07C 275/28,** C07C 233/07, C07C 327/44, C07C 271/44, C07C 279/28, A61K 31/17, A61K 31/27, A61K 31/165, A61K 31/155

(54) **Anti-atherosclerotic diaryl compounds.**

Divisional application 91107475.5 filed on 20/11/89.

(30) Priority : **21.11.88 GB 8827152**

(43) Date of publication of application : **30.05.90 Bulletin 90/22**

(45) Publication of the grant of the patent : **29.07.92 Bulletin 92/31**

(84) Designated Contracting States : **AT BE CH DE ES FR GB GR IT LI LU NL SE**

(56) References cited :
**EP-A- 0 017 484**
**EP-A- 0 283 742**
**EP-A- 0 297 610**
**WO-A-84/02704**
**DE-A- 2 340 874**
**US-A- 4 269 829**
**US-A- 4 387 106**
**US-A- 4 603 145**
**CHEMICAL ABSTRACTS, vol. 98, 1983, page 585, abstract no. 215869z, Columbis, Ohio, US; P. YATES et al.: "Synthetic approachesto haplophytine. 1. Synthesis of 1-methoxy- and 1-hydroxy-6-phenyl-6-(2-pivaloylamidophenyl)non-8-ene-4,5-dione and1-methyl-3-phenyl-3-(2-pivaloylamidophenyl)-2-pyrrolidinone", & CAN. J. CHEM. 1983, 61(3), 509-18**
**CHEMICAL ABSTRACTS, vol. 83, 1975, page 795, abstract no. 9602u, Columbus, Ohio, US; B.F. BOWDEN et al.: "Synthesis of9,10-dihydrophenanthrenes including orchinol methyl ether", & AUST. J. CHEM. 1975, 28(1), 65-80**
**JOURNAL OF ORGANIC CHEMISTRY, vol. 46, no. 22, 1981, pages 4416-4422, American Chemical Society; H. HORINA et al.: "Orthovinylation of aromatic amides via cyclopalladation complexes"**

(73) Proprietor : **THE WELLCOME FOUNDATION LIMITED**
**Unicorn House 160 Euston Road**
**London NW1 2BP (GB)**

(72) Inventor : **Jackson, William Paul**
**The Wellcome Research Laboratories Langley Court**
**Beckenham Kent (GB)**
Inventor : **Harris, Clifford John**
**The Wellcome Research Laboratories Langley Court**
**Beckenham Kent (GB)**
Inventor : **Arrowsmith, Richard James**
**The Wellcome Research Laboratories Langley Court**
**Beckenham Kent (GB)**
Inventor : **Dann, John Gordon**
**The Wellcome Research Laboratories Langley Court**
**Beckenham Kent (GB)**
Inventor : **O'Connor, Kevin Julian**
**The Wellcome Research Laboratories Langley Court**
**Beckenham Kent (GB)**
Inventor : **Booth, Robert Frederick Geoffrey**
**The Wellcome Research Laboratories Langley Court**
**Beckenham Kent (GB)**

(74) Representative : **Garrett, Michael et al**
**The Wellcome Research Laboratories Group Patents and Agreements Langley Court**
**Beckenham Kent BR3 3BS (GB)**

EP 0 370 740 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

(56) References cited :

**CHEMICAL ABSTRACTS, vol. 97, 1982, page 539, abstract no. 38593m, Columbus, Ohio US; A. ALBEROLA et al.: "Alkylaluminuris.XXVI. Reaction of allylic and benzylic ethers with organoaluminum compounds", & AN. QUIM. SER. C. 1982, 78(1), 57-66**

**J.C.S. CHEM. COMM., 1979, pages 499-450; P. de MAYO et al.: "Photochemical synthesis: Quinolines"**

**CHEM. BER., vol. 105, 1972, pages 1634-1645; R.R. SCHMIDT et al.: "Synthese von Heterocyclen durch Thermolyse vono-substituierten aromatischen Diazonium-Ionen"**

**CHEM. BER., vol. 96, 1963, pages 765-770; W.D. ZAHLER et al.: "Intramolekulare Arylierungen in der Diphenylmethan-Reide"**

**COLLECTION CZECHOSLOV. CHEM. COMMUN., vol. 33, 1968, pages 1880-1894; K. PELZ et al.: "Neurotrope und Psychotrope SubstanzenXXIV"**

**J. MEDICINAL CHEMISTRY, vol. 13, January 1970, pages 35-39; W.J. VAN DER BURG et al.: "A novel type of subtituted piperazinewith high antiserotonin potency"**

## Description

The present invention is concerned with new diaryl compounds, processes for their preparation, compositions containing them and their use in medicine, particularly in the prophylaxis or treatment of atherosclerosis.

Intracellular cholesterol ester metabolism in the arterial wall is handled by a variety of enzymes including acyl coenzyme A : cholesterol acyl transferase (ACAT), cholesteryl ester hydrolase (CEH), acid cholesterol hydrolase and cholesteral esterase. Of these, ACAT and cholesteryl ester hydrolase appear to control the steady state concentration of cholesterol ester in the arterial wall:

$$\text{Cholesterol} \underset{\text{CEH}}{\overset{\text{ACAT}}{\rightleftharpoons}} \text{Cholesterol ester}$$

ACAT may also play a key role in the gastrointestinal absorption of cholesterol on the basis that (a) more than 90% of the cholesterol which appears in the lymph is esterified, (b) substantial ACAT activity has been observed in the intestinal mucosal cells of several animal species, (c) the site of greatest intestinal ACAT activity is the jejunum where the majority of cholesterol absorption occurs, (d) ACAT activity in the jejunum parallels increases in dietary cholesterol, and (e) ACAT activity is significantly enhanced in animals having experimental atherosclerosis and in atherosclerotic human tissue and cell cultures.

Compounds having ACAT-inhibitory activity are known from US Patent 4,603,145 and European Patent Application 0297610. USP '145 describes novel diarylalkanamides useful as pharmaceutical agents for ameliorating atherosclerosis by inhibiting the formation and development of atherosclerotic lesions in the arterial wall of mammals. EPA '610 describes substituted urea, thiourea, carbamate and thiocarbamate derivatives which are potent inhibitors of ACAT and are thus useful agents for inhibiting the intestinal absorption of cholesterol.

The following references describe bridged diaryl compounds, some of which are reported to have therapeutic utility in the treatment of conditions which are not associated with ACAT, for example, depression and hypertension:

DE-A-2340874
EP-A-0017484
W0-A-84/02704
Chem. Abs. 98:215869z
Chem. Abs. 83:9602u
J.Org. Chem. 46, 4416 (1981)
Chem. Abs. 97:38593m
JCS Chem. Comm. 1979, 499
Chem. Ber. 105, 1634 (1972)
Chem. Ber. 96, 765 (1963)
Coll. Czech. Chem. Comm. 33, 1880 (1968)
J. Med. Chem. 13, 35 (1970)

We have now discovered a novel class of bridged diaryl compounds which exhibit ACAT inhibitory activity and which are particularly useful as hypolipidaemics as well as for decreasing the steady state concentration of cholesterol and cholesterol ester in the arterial wall and thereby retarding the build-up of atherosclerotic lesions.

According to the present invention, therefore, there are provided compounds of formula (I)

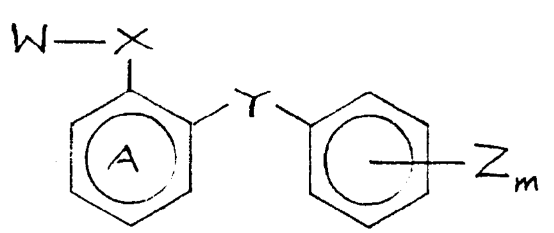

(I)

wherein

m is 0 or 1;

W is a $C_{5-16}$ straight, branched, or cyclic alkyl, alkenyl or alkynyl group, or

$Ph(CH_2)_n$- where Ph is phenyl and n is an integer of from 0 to 2, the phenyl group being optionally substituted by one or more atoms or groups independently selected from halogen, hydroxy, nitro, $C_{1-4}$ alkoxy and $C_{1-4}$ alkyl wherein one or more of the hydrogen atoms in said alkyl group is optionally replaced by halogen, or

$R^1NHCO$- where $R^1$ is hydrogen or a $C_{1-6}$ alkyl group, or

$R^2CONH$- where $R^2$ is hydrogen or a $C_{1-6}$ alkyl group;

X is

$$-(CH_2)_pNHCONH,$$

where p is an integer of from 0 to 2,

$$-NHCOCH_2,$$

$$-NHCOO,$$

$$-CSNH,$$

$$-NHC(:NCN)NH,$$

or

$$-CH(A)CONH$$

where A is halogen;

Y is $-(CH_2)_q-$, where q is an integer of from 1 to 3, or -CH=CH- (E or Z);

Z is a $C_{1-6}$ alkyl group optionally substituted by one or more independently selected polar groups; and

ring A is optionally substituted by one or more fluorine atoms; provided

(i) that in said compound of formula (I) $q \neq 1$ when W = unsubstituted Ph and n = 0, and

(ii) that said compound of formula (I) is not 1-[2,4-difluoro-6-{2-[4-(2,2-dimethylpropyl)phenyl]ethyl}phenyl]-3-heptyl-urea.

and salts or solvates thereof.

The term "cyclic group" as used herein includes groups wherein the cyclic moiety is located at the end of or along the length of a straight or branched alkyl, alkenyl, or alkynyl group, for example, $-CH_2CH(C_6H_{11})CH_3$.

The compound of proviso (ii) is the subject of a divisional application.

Salts of compounds of formula (I) suitable for use in medicine are those which are physiologically acceptable. However, non-physiologically acceptable salts are within the scope of the present invention for use as intermediates in the preparation of the compounds of the invention and their physiologically acceptable salts or solvates.

Preferred compounds of formula (I) having particularly good ACAT inhibiting properties include those wherein

W is $C_{7-11}$ straight alkyl, cyclohexyl, cyclohexylmethyl, or 1,1-dimethylprop-2-ynyl, or

phenyl or benzyl, or

n-$C_4H_9NHCO$-, or

n-$C_3H_7CONH$-;

X is

$$-(CH_2)_pNHCONH,$$

where p is an integer of from 0 to 2,

$$-NHCONHCH_2,$$

$$-NHCOCH_2,\quad -NHCOO,\quad -CSNH,\quad -NHC(:NCN)NH,$$

or

$$-CH(Br)CONH;$$

Z is hydrogen (ie m is O) or, when m is 1, p-isobutyl or p-neopentyl; and
ring A is unsubstituted or disubstituted at the 2,4- or 3,5-positions by fluorine atoms;
and physiologically acceptable salts and solvates thereof.

Particularly preferred compounds of formula (I) having exceptionally desirable ACAT inhibiting properties include those wherein
W is n-heptyl;
X is

$$-NHCONH,\ -NHCOCH_2,$$

or

$$-NHCOO;$$

Y is $-(CH_2)_2-$;
m is 1 and Z is p-isobutyl or p-neopentyl; and
ring A is disubstituted at the 2,4-positions by fluorine atoms;
and physiologically acceptable salts or solvates thereof.

According to further aspects of the invention, there are also provided:

(a) compounds of formula (I) and pharmaceutically acceptable salts or solvates thereof for use in therapy;

(b) pharmaceutical formulations comprising a compound of formula (I) and/or one of its pharmaceutically acceptable salts or solvates and at least one pharmaceutical carrier or excipient;

(c) the use of a compound of formula (I) or of a pharmaceutically acceptable salt or solvate in the manufacture of a medicament for the prophylaxis or treatment of a clinical condition for which an ACAT inhibitor is indicated;

(d) processes for the preparation of compounds of formula (I) (including salts and solvates thereof).

With regard to aspects (a) and (c), the ability of compounds of the formula (I) to inhibit ACAT activity renders them useful as hypolipidaemics and in reducing the steady state concentration of cholesterol and cholesterol ester in the arterial wall, thereby retarding the build-up of atherosclerotic lesions and providing application for these compounds in the prophylaxis or treatment of atherosclerosis.

Hereinafter all references to "compound(s) of formula (I)" refer to compound(s) of formula (I) as defined above including their salts, and solvates.

The amount of a compound of formula (I) which is required to achieve the desired biological effect will, of course, depend on a number of factors, for example, the specific compound chosen, the use for which it is intended, the mode of administration, and the clinical condition of the recipient. In general, a daily dose is expected to lie in the range of from 1ng to 100mg, typically from 50ng to 50mg, per day per kilogram bodyweight, for example 500ng-5mg/kg/day. An intravenous dose may, for example, be in the range of from 10ng to 1 mg/kg which may conveniently be administered as an infusion of from 0.1ng to 50 g per kilogram per minute. Infusion fluids suitable for this purpose may contain, for example, from 0.01ng to 100 g, typically from 0.1ng to 100 g, per millilitre. Unit doses, for example, may contain from 100ng to 100mg of the active compound; for example, ampoules for injection may contain from 100ng to 1mg and orally administrable unit dose formulations, such as tablets or capsules, may contain, for example, from 0.001 to 50mg, typically from 0.02 to 20mg. In the case of physiologically acceptable salts, the weights indicated above refer to the weight of the diaryl ion derived from the salt.

For the prophylaxis or treatment of the conditions referred to above, the compounds of formula (I) may be used as the compound per se, but are preferably presented with an acceptable carrier as a pharmaceutical formulation. The carrier must, of course, be acceptable in the sense of being compatible with the other ingredients of the formulation and not be deleterious to the recipient thereof. The carrier may be a solid or a liquid, or both, and is preferably formulated with the compound as a unit-dose formulation, for example, a tablet, which may contain from 0.05% to 95% by weight of active compound. Other pharmacologically active substances may also be present in formulations of the present invention. One or more of the compounds of formula (I) may be incorporated in the formulations of the invention, which may be prepared by any of the well known techniques of pharmacy consisting essentially of admixture of the components.

The formulations include those suitable for oral, rectal, topical buccal (e.g. sub-lingual), and parenteral (e.g. subcutaneous, intramuscular, intradermal, or intravenous) administration, although the most suitable route in any given case will depend on the nature and severity of the condition being treated and on the nature of the

particular compound of formula (I), or the physiologically acceptable salt thereof, which is being used.

Formulations suitable for oral administration may be presented in discrete units, such as capsules, cachets, lozenges or tablets, each containing a predetermined amount of a compound of formula (I) or a physiologically acceptable salt thereof; as a powder or granules; as a solution or a suspension in an aqueous or non-aqueous liquid; or as an oil-in-water or water-in-oil emulsion. Such formulations may be prepared by any suitable method of pharmacy which includes the step of bringing into association the active compound and the carrier (which may constitute one or more accessory ingredients). In general, the formulations are prepared by uniformly and intimately admixing the active compound with a liquid or finely divided solid carrier or both, and then, if necessary, shaping the product. For example, a tablet may be prepared by compressing or moulding a powder or granules of the compound, optionally with one or more accessory ingredients. Compressed tablets may be prepared by compressing, in a suitable machine, the compound in a free-flowing form, such as a powder or granules optionally mixed with a binder, lubricant, inert diluent, and/or surface active/dispersing agent(s). Moulded tablets may be made by moulding, in a suitable machine, the powdered compound moistened with an inert liquid diluent.

Formulations suitable for buccal (sub-lingual) administration include lozenges comprising a compound of formula (I), or a physiologically acceptable salt thereof, in a flavoured base, usually sucrose and acacia or tragacanth; and pastilles comprising the compound in an inert base such as gelatin and glycerin or sucrose and acacia.

Formulations of the present invention suitable for parenteral administration conveniently comprise sterile aqueous preparations of a compound of formula (I), or a physiologically acceptable salt thereof, which preparations are preferably isotonic with the blood of the intended recipient. These preparations are preferably administered intravenously, although administration may also be effected by means of subcutaneous, intramuscular, or intradermal injection. Such preparations may conveniently be prepared by admixing the compound with water and rendering the resulting solution sterile and isotonic with the blood. Injectable compositions according to the invention will generally contain from 0.1 to 5% w/w of active compound.

Formulations suitable for rectal administration are preferably presented as unit-dose suppositories. These may be prepared by admixing a compound of formula (I), or a physiologically acceptable salt thereof, with one or more conventional solid carriers, for example, cocoa butter, and then shaping the resulting mixture. Formulations suitable for topical application to the skin preferably take the form of an ointment, cream, lotion, paste, gel, spray, aerosol, or oil. Carriers which may be used include vaseline, lanoline, polyethylene glycols, alcohols, and combinations of two or more thereof. The active compound is generally present at a concentration of from 0.1 to 15% w/w of the composition, for example, from 0.5 to 2%.

The compounds of formula (I) may be prepared in any conventional manner, for example, by the process described below. According to this aspect of the invention, compounds of formula (I) may be prepared by reacting a compound of formula (II)

W - P (II)

wherein W is as hereinbefore defined and P is as defined below, with a compound of formula (III)

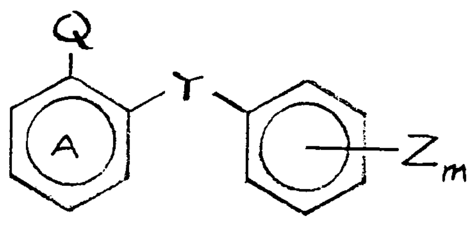

(III)

wherein m, Y and Z are as hereinbefore defined, ring A is optionally substituted as hereinbefore defined and Q is a nucleophilic group, for example, amino or hydroxy, capable of reacting with the group P, for example, isocyanate, in compound (II) or, alternatively, P in compound (II) is a nucleophilic group, for example, amino, capable of reacting with the group Q, for example, $-CH_2CO_2H$, in compound (III) to give a compound of formula (I). The reaction is typically carried out in a non-polar solvent, such as THF, in the presence of a suitable base, for example, DMAP.

Compounds of formula (III) may be prepared by reacting a compound of formula (IV)

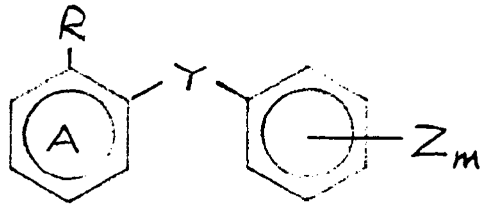

(IV)

wherein m, Y and Z are as hereinbefore defined, ring A is optionally substituted as hereinbefore defined, and R is a group capable of being converted to the group Q of compound (III), with a suitable reagent or reagents and under such conditions as to effect conversion of R to Q, for example, converting a nitro group to an amino group by catalytic hydrogenation.

compounds of formula (IV) may be prepared by selectively reducing the carbonyl group of a compound of formula (V)

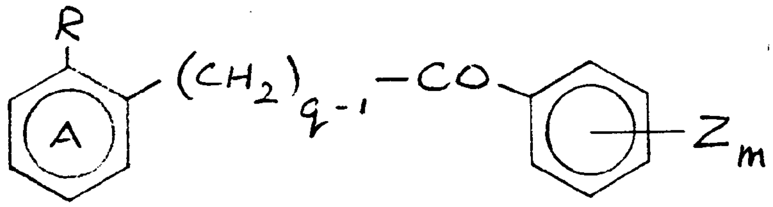

(V)

wherein m, q, R and Z are as hereinbefore defined and ring A is optionally substituted as hereinbefore defined, using, for example, triethylsilane in the presence of trifluoroacetic acid.

Compounds of formula (V) may be prepared by reacting a compound of formula (VI)

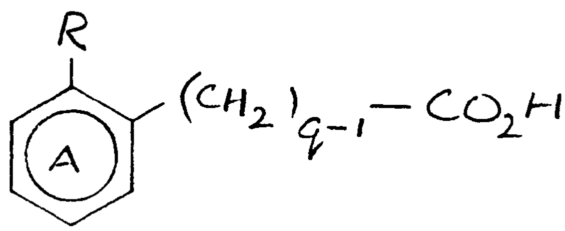

(VI)

wherein R and q are as hereinbefore defined and ring A is optionally substituted as hereinbefore defined, with a compound of formula (VII)

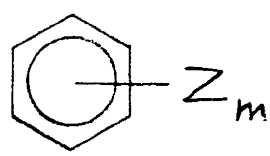

(VII)

wherein m and Z is as hereinbefore defined, using, for example, Friedel-Crafts acylation conditions.

Compounds of formula (VI) may be prepared by reacting a compound of formula (VIII)

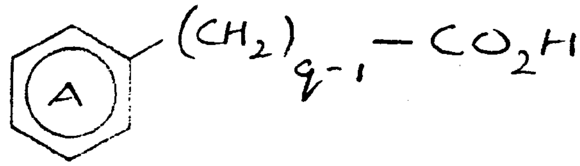

(VIII)

wherein q is as hereinbefore defined and ring A is optionally substituted as hereinbefore defined, with a suitable reagent or reagents and under such conditions as to substitute the group R of compound (VI) in the o-position, for example, in the case where R is nitro, by selective nitration using c.$H_2SO_4$/c.$HNO_3$ at low temperature.

Compounds of formulae (II) and (VII) are commercially available or may be prepared by methods well known to those skilled in the art or readily available from the chemical literature.

Optional conversion of a compound of formula (I) to a corresponding salt may be effected by reaction with the appropriate acid or base.

For a better understanding of the invention, the following Examples are given by way of illustration.

Compositive Example

Preparation of 1-[2,4-difluoro-6-{2-[4-(2,2-dimethylpropyl)phenyl]ethyl}phenyl]-3-heptylurea

(a) 2-Nitro-3,5-difluorophenyl acetic acid

A solution of 3,5-difluorophenylacetic acid (20g, Fluorochem) in c.$H_2SO_4$ (150ml) was stirred at -15°C and c.$HNO_3$ (75ml) added dropwise over 1 hour at a temperature of from -5 to -10°C. The mixture was stirred for a further 1/2 hour at -10°C and then poured into ice-water (1.0 l). The latter was extracted with ether (x3) and the combined extracts washed with water (x2), dried over $MgSO_4$, and evaporated under reduced pressure to give a solid which was crystallised and then recrystallised from ether/hexane (1:4 v/v) to give 20.8g of the desired product.

Analysis:

Calcd C 44.24; H 2.30; N 6.45

Found C 44.18; H 2.38; N 6.24

200MHz $^1H$ NMR consistent with proposed structure.

(b) 2.4-difluoro-6-[4-(2,2 -dimethylpropyl)benzoylmethyl]-1-nitrobenzene

A suspension of the product from step (a) (10g) in neopentyl benzene (40ml, Fluka) was stirred at room temperature and oxalyl chloride (8.8g) was added dropwise over 5 minutes, then DMF (5-10 drops). The mixture was stirred overnight at room temperature, then evaporated under reduced pressure to give an orange/yellow solution to which $AlCl_3$ (7.4g) was added portionwise over 15 minutes. The resulting mixture was stirred for 1/2 hour at room temperature, followed by 4 hours at 60°C, then poured into ice/c.HCl (300g/50ml). The latter was extracted with ethyl acetate (x3) and the combined extracts washed with 2N HCl, 2N aqu. NaOH (x2), 2N HCl, and water (x2), dried over $MgSO_4$/charcoal, and evaporated under reduced pressure to give a brown oil which was flash chromatographed through a silica column using ether/hexane (3:7 v/v). The eluate was evaporated under reduced pressure and the residue crystallised from ether/hexane (1:3 v/v) to give 6.3g of the desired product.

Analysis:

Calcd C 65.72; H 5.54; N 3.99

Found C 65.71; H 5.48; N 4.03

200MHz $^1H$ NMR consistent with proposed structure.

(c) 2,4-Difluoro-6-{2-[4-(2,2-dimethylpropyl)phenyl]ethyl}-1-nitrobenzene

A solution of the product from step (b) (11.1g) in trifluoroacetic acid (16ml) was stirred at room temperature and triethylsilane (12.5ml) added. The mixture was stirred overnight at room temperature, followed by 3 hours at 50°C during which further portions of triethylsilane (3ml each) were added after 1.5 hours and 2.5 hours, then poured into water (250ml). The latter was extracted with ether (x2) and the combined extracts washed with water (x2), dried over $MgSO_4$, and evaporated under reduced pressure to give an oil which was flash chromatographed through a silica column using ether/hexane (1:4 v/v). The eluate was evaporated under reduced pressure to give 11.0g of the desired product.

Analysis:

Calcd 68.47; H 6.31; N 4.20

Found 68.75; H 6.75; N 4.24

200MHz $^1H$ NMR consistent with proposed structure.

(d) 2,4-Difluoro-6-{2-[4-(2,2-dimethylpropyl)phenyl]ethyl}aniline

10% Pd/C (600mg) was added under nitrogen to a stirred solution of the product from step (c) (10.9g) in ethanol (150ml) and the mixture hydrogenated at room temperature at a pressure of 1 atm.

$H_2$ for 3.5 hours (uptake 2300ml). The mixture was filtered, the residue washed with ethanol, and the filtrate evaporated under reduced pressure to give an oil which crystallised on standing to give 8.0g of the desired product.

Analysis:

Calcd C 75.25; H 7.59; N 4.62

Found C 75.61; H 8.01; N 4.62

200 MHz $^1$H NMR consistent with proposed structure.

(e) 1-[2,4-Difluoro-6-{2-[4-(2,2-dimethylpropyl)phenyl]ethyl}phenyl]3-heptylurea

A solution of the product from step (d) (4.5g) in THF (50ml) was stirred at room temperature and heptyl isocyanate (5.5g containing 36 mole % benzene, prepared by the method described in Organic Syntheses, collective Volume 3, p.846) and N,N-dimethylaminopyridine (750mg) were added. The mixture was stirred at room temperature for ca 60 hours, then evaporated under reduced pressure to give an oil which was flash chromatographed through a silica column using ether/hexane (2:1 v/v). The eluate was evaporated under reduced pressure and the residue crystallised from hexane and triturated with methanol to give 4.5g of material. The latter was flash chromatographed through a silica column using ether/hexane (2:1 v/v) and then freeze-dried from dioxan to give 5.3g of the desired product, mp 105-107°C.

Analysis:

Calcd C 72.97; H 8.56; N 6.31

Found C 72.16; H 8.45; N 6.17

200MHz $^1$H NMR consistent with proposed structure.

Synthetic Examples 1-28

The following compounds of formula (I) were prepared in a manner analogous to the method of the Compositive Example.

1) N-Heptyl-N′-{2-[2-(4-isobutylphenyl)ethyl]phenyl}urea, mp 105-107°C;
2) N-[2-{2-[4-(2,2-Dimethylpropyl)phenyl]ethyl}phenyl]-N′-heptylurea, colourless foam;
3) N-Heptyl-N′-{2-[3-(4-isobutylphenyl)propyl]phenyl}urea, mp 79-82°C;
4) N-Heptyl-N′-2-(4-isobutylphenylmethyl)phenylurea, mp 107-109°C;
5) N-Heptyl-N′-[2-(4-isobutylphenylvinyl)phenyl]urea, mp 86°C (softens 78°C);
6) N-{2-[2-(4-Isobutylphenyl)ethyl]phenyl-N-pentylurea, mp 105-106°C;
7) N-Decyl-N′-{2-[2-(4-isobutylphenyl)ethyl]phenyl}urea, mp 105-107°C;
8) N-Cyclohexanemethyl-N′-{2-[2-(4-isobutylphenyl)ethyl]phenyl}urea, mp 137-139°C;
9) N-{2-[2-(4-Isobutylphenyl)ethyl]phenyl}-2-bromodecanamide, mp 96-97°C;
10) N-{2-[2-(4-Isobutylphenyl)ethyl]phenyl}nonanethioamide, mp 34-36°C;
11) N-Heptyl-2-[2-(4-isobutylphenyl)ethyl]phenylacetamide, mp 50-52°C;
12) N-2-[2-(4-Isobutylphenyl)ethyl]phenyloxycarbonylheptylamine, mp 55-57°C;
13) N-Heptyl-N′-{2-[2-(4-isobutylphenyl)ethyl]-4,6-difluorophenyl}urea, mp 100-102°C;
14) N-Cyclohexyl-N′-{2-[2-(4-isobutylphenyl)ethyl]phenyl}urea, mp 143-145°C;
15) N-{2-[2-(4-Isobutylphenyl)ethyl]phenyl}-N′-nonylurea, mp 104-107°C;
16) N-{2-[2-(4-Isobutylphenyl)ethyl]phenyl}-N′-undecylurea, mp 98-100°C;
17) N-Benzyl-N′-{2-[2-(4-isobutylphenyl)ethyl]phenyl)urea, mp 153-154°C;
18) N-{2-[2-(4-Isobutylphenyl)ethyl]phenyl}-N′-phenylurea, mp 161-163°C;
19) N-[(N-1-Butylcarbamoyl)methyl]-N′-{2-[2-(4-neopentylphenyl)ethyl--4,6-difluoro]phenyl}urea, mp 160-161°C;
20) N-[(N-1-Butylcarbamoyl)methyl]-N′-{2-[2-(4-neopentylphenyl)ethyl]phenyl}urea, mp 146-147°C;
21) N-(2-Butyramidoethyl)-N′-{2-[2-(4-neopentylphenyl)ethyl-4,6-difluoro]phenyl}urea, no mp (lyophilisate);
22) N-{2-[2-(4-Isobutylphenyl)ethyl]benzyl-N′-hexylurea, mp 95°C;
23) N-[2,4-difluoro-6-(2-phenylethyl)phenyl]-N′-heptylurea, mp 111-113°C;
24) 2-cyano-1-heptyl-3-{2-[2-(4-isobutylphenyl)ethyl]phenyl}guanidine, mp 82-84°C;
25) {2,4-Difluoro-6-[4-(2,2-dimethylpropyl)benzyl]phenyl}-N-heptyl-carbamate, mp 58-59°C;
26) {2,4-Difluoro-6-{2-[4-(2,2-dimethylpropyl)phenyl]-1-ethyl}-phenyl}-N-heptylcarbamate, mp 55-56°C;
27) 2-Bromo-N-{3,5-difluoro-2-[2-(4-neopentylphenyl)ethyl]penyl} canamide, mp 76-78°C; and
28) 1-{2,4-Difluoro-6-[2-(4-neopentylphenyl)ethyl}-3-(1,1-dimethylprop-2-ynyl)urea, mp 145-148°C.

The NMR's, IR's and elemental analyses of the compounds of Examples 1 to 28 were all consistent with

the proposed structures.

Pharmaceutical Formulation Examples

In the following Examples, the "active ingredient" is any compound of formula (I) as hereinbefore defined, preferably one of the compounds of Synthetic Examples 1 to 28.

(i) Tablet formulations

The following formulations A, B and C may be prepared by wet granulation of ingredients (a) to (c), (a) to (d) and (a) to (c) respectively with a solution of povidone, followed by addition of the magnesium stearate and compression.

Formulation A

| | | mg/tablet | mg/tablet |
|---|---|---|---|
| (a) | Active ingredient | 250 | 250 |
| (b) | Lactose B.P. | 210 | 26 |
| (c) | Sodium Starch Glycollate | 20 | 12 |
| (d) | Povidone B.P. | 15 | 9 |
| (e) | Magnesium Stearate | 5 | 3 |
| | | 500 | 300 |

Formulation B

| | | mg/tablet | mg/tablet |
|---|---|---|---|
| (a) | Active ingredient | 250 | 250 |
| (b) | Lactose 150 | 150 | - |
| (c) | Avicel PH 101 | 60 | 26 |
| (d) | Sodium Starch Glycollate | 20 | 12 |
| (e) | Povidone B.P. | 15 | 9 |
| (f) | Magnesium Stearate | 5 | 3 |
| | | 500 | 300 |

Formulation C

| | | mg/tablet |
|---|---|---|
| (a) | Active ingredient | 100 |
| (b) | Lactose | 200 |
| (c) | Starch | 50 |
| (d) | Povidone | 5 |
| (e) | Magnesium Stearate | 4 |
| | | 359 |

The following formulations D and E may be prepared by direct compression of the admixed ingredients. The lactose used in formulation E is of the direct compression type.

Formulation D

| | mg/capsule |
|---|---|
| Active ingredient | 250 |
| Magnesium Stearate | 4 |
| Pregelatinised Starch NF15 | 146 |
| | 400 |

Formulation E

| | mg/capsule |
|---|---|
| Active ingredient | 250 |
| Magnesium Stearate | 5 |
| Lactose | 145 |
| Avicel | 100 |
| | 500 |

Formulation F (Controlled release formulation)

| | | mg/tablet |
|---|---|---|
| (a) | Active ingredient | 500 |
| (b) | Hydroxypropylmethylcellulos (Methocel K4M Premium) | 112 |
| (c) | Lactose B.P. | 53 |
| (d) | Povidone B.P.C. | 28 |
| (e) | Magnesium Stearate | 7 |
| | | 700 |

The formulation may be prepared by wet granulation of ingredients (a) to (c) with a solution of povidone, followed by addition of the magnesium stearate and compression.

(ii) Capsule formulations

Formulation A

Capsules may be prepared by admixing the ingredients of Formulation D above and filling two-part hard gelatin capsules with the resulting mixture. Formulation B (infra) may be prepared in a similar manner.

Formulation B

| | | mg/capsule |
|---|---|---|
| (a) | Active ingredient | 250 |
| (b) | Lactose B.P. | 143 |
| (c) | Sodium Starch Glycollate | 25 |
| (d) | Magnesium Stearate | 2 |
| | | 420 |

Formulation C

| | | mg/capsule |
|---|---|---|
| (a) | Active ingredient | 250 |
| (b) | Macrogel 4000 BP | 350 |
| | | 600 |

Capsules may be prepared by melting the Macrogel 4000 BP, dispersing the active ingredient in the melt, and filling two-part hard gelatin capsules therewith.

Formulation D

| | mg/capsule |
|---|---|
| Active ingredient | 250 |
| Lecithin | 100 |
| Arachis Oil | 100 |
| | 450 |

Capsules may be prepared by dispersing the active ingredient in the lecithin and arachis oil and filling soft, elastic gelatin capsules with the dispersion.

Formulation E (Controlled release capsule)

| | | mg/capsule |
|---|---|---|
| (a) | Active ingredient | 250 |
| (b) | Microcrystalline Cellulose | 125 |
| (c) | Lactose BP | 125 |
| (d) | Ethyl Cellulose | 13 |
| | | 513 |

The controlled-release capsule formulation may be prepared by extruding mixed ingredients (a) to (c) using an extruder, then spheronising and drying the extrudate. The dried pellets are coated with ethyl cellulose (d) as a controlled-release membrane and filled into two-part hard gelatin capsules.

(iii) Intravenous injection formulation

Active ingredient 0.200g

Sterile, pyrogen-free phosphate buffer (pH 9.0) to 10 ml

The active ingredient is dissolved in most of the phosphate buffer at 35-40°C, then made up to volume and filtered through a sterile micropore filter into sterile 10 ml glass vials (Type 1) which are sealed with sterile closures and overseals.

(iv) Intramuscular injection formulation

| | | |
|---|---|---|
| Active ingredient | | 0.20 g |
| Benzyl Alcohol | | 0.10 g |
| Glycofurol 75 | | 1.45 g |
| Water for Injection | q.s. to | 3.00 ml |

The active ingredient is dissolved in the glycofurol. The benzyl alcohol is then added and dissolved, and water added to 3 ml. The mixture is then filtered through a sterile micropore filter and sealed in sterile 3 ml glass vials (Type 1).

(v) Syrup formulation

| | | |
|---|---|---|
| Active ingredient | | 0.2500 g |
| Sorbitol Solution | | 1.5000 g |
| Glycerol | | 1.0000 g |
| Sodium Benzoate | | 0.0050 g |
| Flavour | | 0.0125 ml |
| Purified Water | q.s. to | 5.0 ml |

The sodium benzoate is dissolved in a portion of the purified water and the sorbitol solution added. The active ingredient is added and dissolved. The resulting solution is mixed with the glycerol and then made up to the required volume with the purified water.

(vi) Suppository formulation

| | mg/suppository |
|---|---|
| Active ingredient (63μm)* | 250 |
| Hard Fat, BP (Witepsol H15 - Dynamit Nobel) | 1770 |
| | 2020 |

* The active ingredient is used as a powder wherein at least 90% of the particles are of 63μm diameter or less.

One-fifth of the Witepsol H15 is melted in a steam-jacketed pan at 45°C maximum. The active ingredient is sifted through a 200μm sieve and added to the molten base with mixing, using a Silverson fitted with a cutting head, until a smooth dispersion is achieved. Maintaining the mixture at 45°C, the remaining Witepsol H15 is added to the suspension which is stirred to ensure a homogenous mix. The entire suspension is then passed through a 250μm stainless steel screen and, with continuous stirring, allowed to cool to 40°C. At a temperature of 38-40°C, 2.02g aliquots of the mixture are filled into suitable plastic moulds and the suppositories allowed to cool to room temperature.

(vii) Pessary formulation

| | mg/pessary |
|---|---|
| Active ingredient (63μm) | 250 |
| Anhydrous Dextrose | 380 |
| Potato Starch | 363 |
| Magnesium Stearate | 7 |
| | 1000 |

The above ingredients are mixed directly and pessaries prepared by compression of the resulting mixture.

Biological assay

In vitro inhibition of ACAT

The in vitro esterification of cholesterol in the presence of ACAT and the test compound was assayed radiometrically using [$^{14}$C]oleoyl CoA as substrate:

$$[^{14}C]\text{oleoyl CoA} + \text{cholesterol} \longrightarrow [^{14}C]\text{oleoyl cholesterol} + \text{CoASH}$$

The enzyme is membrane-associated in vivo. Microsomal protein is therefore used as the source of both ACAT and cholesterol. The compounds of the invention were tested against enzyme derived from human embryo 407 intestinal epithelial cell line.

[$^{14}$C]Oleoyl CoA was incubated with microsomal protein at 37°C, pH 7.0, in the presence of various concentrations of the test compound. After 4 minutes, the reaction was stopped by the addition of ice-cold chloroform/methanol containing a known amount of [$^{3}$H]oleoyl cholesterol to compensate for the loss of any [$^{14}$C] product. A known volume of the resulting lower phase, which contains lipidic material from the reaction, was dried, redissolved in hexane containing unlabelled oleoyl cholesterol (TLC marker), and run on a quantitative TLC plate (silica gel). The oleoyl cholesterol spot was visualised (iodine vapour), removed from the TLC plate, and its radioactivity measured by scintillation counting.

A plot of ACAT inhibitory activity vs concentration was prepared for each test compound and the corresponding $IC_{50}$ determined. The compounds of Synthetic Examples 1 to 28 where all found to significantly inhibit ACAT.

## Claims

**Claims for the following Contracting States : AT, BE, CH, DE, FR, GB, IT, LI, LU, NL, SE**

1. A compound of formula (I)

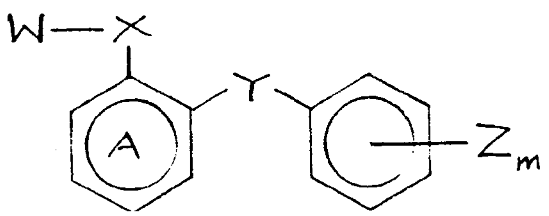

(I)

wherein
m is 0 or 1;
W is a $C_{5-16}$ straight, branched, or cyclic alkyl, alkenyl or alkynyl group, alkenyl or alkynyl, or

Ph$(CH_2)_n$- where Ph is phenyl and n is an integer of from 0 to 2, the phenyl group being optionally substituted by one or more atoms or groups independently selected from halogen, hydroxy, nitro, $C_{1-4}$ alkoxy and $C_{1-4}$ alkyl wherein one or more of the hydrogen atoms in said alkyl group is optionally replaced by halogen, or

$R^1$NHCO- where $R^1$ is hydrogen or a $C_{1-6}$ alkyl group, or

$R^2$CONH- where $R^2$ is hydrogen or a $C_{1-6}$ alkyl group;

X is

$$-(CH_2)_pNHCONH,$$

where p is an integer of from 0 to 2,

$$-NHCONHCH_2,$$

$$-NHCOCH_2, \quad -NHCOO, \quad -CSNH,$$

$$-NHC(:NCN)NH,$$

or

$$-CH(A)CONH$$

where A is halogen;
Y is $-(CH_2)_q-$, where q is an integer of from 1 to 3, or -CH=CH- (E or Z);
Z is a $C_{1-6}$ alkyl group optionally substituted by one or more independently selected polar groups; and
ring A is optionally substituted by one or more fluorine atoms; provided

(i) that in said compound of formula (I) q ≠ 1 when W = unsubstituted Ph and n = 0, and

(ii) that said compound of formula (I) is not 1-[2,4-difluoro-6-{2-[4-(2,2-dimethylpropyl)phenyl]ethyl}phenyl]-3-heptylurea.

and salts or solvates thereof.

2. A compound of formula (I) as claimed in claim 1, wherein
W is $C_{7-11}$ straight alkyl, cyclohexyl, cyclohexyl-methyl,or 1,1-dimethylprop-2-ynyl, or
phenyl or benzyl, or
<u>n</u>-$C_4H_9NHCO$-, or
<u>n</u>-$C_3H_7CONH$-;
X is

$$-(CH_2)_pNHCONH,$$

where p is an integer of from 0 to 2,

$$-NHCONHCH_2,$$

$$-NHCOCH_2,\quad -NHCOO,\quad -CSNH,\quad -NHC(:NCN)NH,$$

or

$$-CH(Br)CONH;$$

Z is hydrogen (ie m is 0) or, when m is 1, p-isobutyl or p-neopentyl; and
ring A is unsubstituted or disubstituted at the 2,4- or 3,5-positions by fluorine atoms;
and physiologically acceptable salts and solvates thereof.

3. A compound of formula (I) as claimed in claim 2, wherein
W is n-heptyl;
X is

$$-NHCONH,\quad -NHCOCH_2,$$

or

$$-NHCOO;$$

Y is $-(CH_2)_2-$;
m is 1 and Z is <u>p</u>-isobutyl or <u>p</u>-neopentyl; and
ring A is disubstituted at the 2,4-positions by fluorine atoms;
and physiologically acceptable salts or solvates thereof.

4. A pharmaceutical formulation comprising a compound of formula (I) as claimed in claim 1 or a physiologically acceptable salt or solvate thereof, a pharmaceutically acceptable carrier or excipient and, optionally, one or more other pharmacologically active agents.

5. A formulation as claimed in claim 4, wherein the compound of formula (I) is as claimed in claim 2 or is a physiologically acceptable salt or solvate thereof.

6. A formulation as claimed in claim 4, wherein the compound of formula (I) is as claimed in claim 3 or is a physiologically acceptable salt or solvate thereof.

7. A formulation as claimed in any of claims 4 to 6 which is in the form of a tablet or capsule.

8. A process for the preparation of a compound of formula (I) as claimed in claim 1 which comprises reacting a compound of formula (II)

W-P (II)

wherein W is as defined in claim 1, with a compound of formula (III)

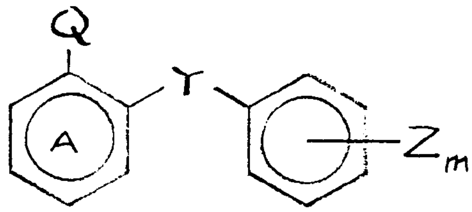

(III)

wherein m, Y and Z are as defined in claim 1 and ring A is optionally substituted as defined in claim 1, either P in the compound of formula (II) or Q in the compound of formula (III) being a nucleophilic group capable of reacting with Q in the compound of formula (III) or, alternatively, P in the compound of formula (II) respectively to give a compound of formula (I) having a linkage X as defined in claim 1;
and optionally converting the compound of formula (I) so formed to a corresponding salt or solvate.

9. A compound of formula (I) as claimed in any of claims 1 to 3 or a physiologically acceptable salt of solvate thereof, for use in therapy.

10. A compound of formula (I) as claimed in any of claims 1 to 3 or a physiologically acceptable salt or solvate thereof, for use in the prophylaxisor treatment of a clinical condition for which an ACAT inhibitor is indicated.

11. A compound of formula (I) as claimed in any of claims 1 to 3 or a physiologically acceptable salt or solvate thereof, for use in the prophylaxis or treatment of atherosclerosis.

12. Use of a compound of formula (I) as claimed in any of claims 1 to 3 or of a physiologically acceptable salt or solvate thereof, in the manufacture of a medicament for the prophylaxis or treatment of a clinical condition for which an ACAT inhibitor is indicated.

13. Use of a compound of formula (I) as claimed in any of claims 1 to 3, or of a physiologically acceptable salt or solvate thereof, in the manufacture of a medicament for the prophylaxis or treatment of atherosclerosis.

**Claims for the following Contracting States : ES, GR**

1. A process for the preparation of a compound of formula (I)

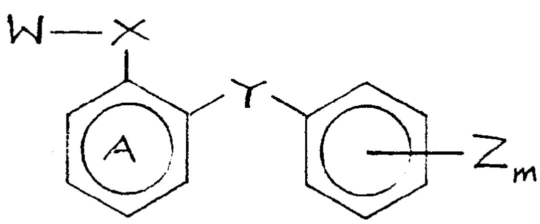

(I)

wherein
m is 0 or 1;
W is a $C_{5-16}$ straight, branched, or cyclic alkyl, alkenyl or alkynyl group, or

$Ph(CH_2)_n$- where Ph is phenyl and n is an integer of from 0 to 2, the phenyl group being optionally substituted by one or more atoms or groups independently selected from halogen, hydroxy, nitro, $C_{1-4}$ alkoxy and $C_{1-4}$ alkyl wherein one or more of the hydrogen atoms in said alkyl group is optionally replaced by halogen, or

$R^1NHCO$- where $R^1$ is hydrogen or a $C_{1-6}$ alkyl group, or

$R^2CONH$- where $R^2$ is hydrogen or a $C_{1-6}$ alkyl group;

X is

$$-(CH_2)_pNHCONH,$$

where p is an integer of from 0 to 2,

$-NHCONHCH_2$,

$-NHCOCH_2$, $-NHCOO$, $-CSNH$,

$-NHC(:NCN)NH$,

or

$-CH(A)CONH$

where A is halogen;
Y is $-(CH_2)_q-$, where q is an integer of from 1 to 3, or -CH=CH- (E or Z);
Z is a $C_{1-6}$ alkyl group optionally substituted by one or more independently selected polar groups; and
ring A is optionally substituted by one or more fluorine atoms;
provided

(i) that in said compound of formula (I) $q \neq 1$ when W = unsubstituted Ph and n = 0,
(ii) that said compound of formula (I) is not 1-[2,4-difluoro-6-{2-[4-(2,2-dimethylpropyl)phenyl]ethyl}phenyl]-3-heptylurea.

which process comprises reacting a compound of formula (II)

W-P (II)

wherein W is as defined in claim 1, with a compound of formula (III)

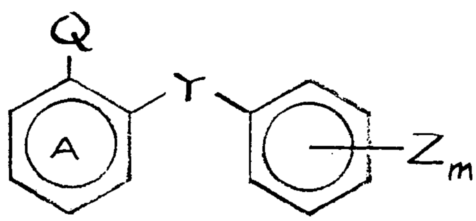

(III)

wherein m, Y and Z are as defined in claim 1 and ring A is optionally substituted as defined in claim 1, either P in the compound of formula (II) or Q in the compound of formula (III) being a nucleophilic group capable of reacting with Q in the compound of formula (III) or, alternatively, P in the compound of formula (II) respectively to give a compound of formula (I) having a linkage X as defined in claim 1;
and optionally converting the compound of formula (I) so formed to a corresponding salt or solvate.

2. A process as claimed in claim 1 for the preparation of a compound of formula (I) as defined in claim 1, wherein
W is $C_{7-11}$ straight alkyl, cyclohexyl, cyclohexylmethyl, or 1,1-dimethylprop-1-ynyl, or
phenyl or benzyl, or
n-$C_4H_9NHCO$-, or
n-$C_3H_7CONH$-;
X is

$-(CH_2)_pNHCONH$,

where p is an integer of from 0 to 2,

$-NHCONHCH_2$,

$-NHCOCH_2$, $-NHCOO$, $-CSNH$, $-NHC(:NCN)NH$,

or

$-CH(Br)CONH$;

Z is hydrogen (ie m is O) or, when m is 1, p-isobutyl or p-neopentyl; and
ring A is unsubstituted or disubstituted at the 2,4- or 3,5-positions by fluorine atoms;

and physiologically acceptable salts or solvates thereof.

3. A process as claimed in claim 2 for the preparation of a compound of formula (I) as defined in claim 1, wherein

W is n-heptyl;

X is

$$-NHCONH, \quad -NHCOCH_2,$$

or

$$-NHCOO;$$

Y is $-(CH_2)_2-$;

m is 1 and Z is p-isobutyl or p-neopentyl; and

ring A is disubstituted at the 2,4-positions by fluorine atoms;

and physiologically acceptable salts or solvates thereof.

4. A process as claimed in claim 1 for the preparation of a compound of formula (I) in which X is -NHCONH, wherein P in the compound of formula (II) is an isocyanate group and Q in the compound of formula (III) is an amino group.

5. A process as claimed in claim 1 for the preparation of a compound of formula (I) in which X is $-NHCOCH_2$, wherein P in the compound of formula (II) is an amino group and Q in the compound of formula (III) is a carboxymethyl group.

6. A process as claimed in claim 1 for the preparation of a compound of formula (I) in which X is -NHCOO, wherein P in the compound of formula (II) is an isocyanate group and Q in the compound of formula (III) is a hydroxy group.

7. A method of preparing a pharmaceutical formulation which comprises

(a) preparing a compound of formula (I) or a physiologically acceptable salt or solvate thereof by a process as claimed in any of claims 1 to 6; and

(b) admixing the product from step (a) with a pharmaceutically acceptable carrier or excipient and, optionally, one or more other pharmacologically active agents.

8. A method as claimed in claim 7 which comprises an additional step (c) wherein the admixture from step (b) is formed into a tablet or capsule.

## Patentansprüche

### Patentansprüche für folgende Vertragsstaaten : AT, BE, CH, DE, FR, GB, IT, LI, LU, NL, SE

1. Verbindung der Formel (I)

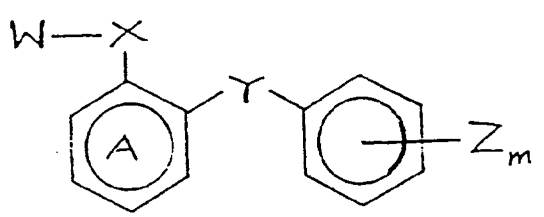 (I),

worin

m Null oder 1 ist,

W eine gerade, verzweigte oder cyclische $C_5$-$C_{16}$-Alkyl-, Alkenyl- oder Alkinylgruppe oder

$Ph(CH_2)_n$, wobei Ph Phenyl darstellt und n eine ganze Zahl von Null bis 2 ist, wobei die Phenylgruppe gegebenenfalls durch ein oder mehrere Atome oder Gruppen unabhängig ausgewählt aus Halogen, Hydroxy, Nitro, $C_1$-$C_4$-Alkoxy und $C_1$-$C_4$-Alkyl substituiert ist, wobei ein oder mehrere der Wasserstoffatome in der Alkylgruppe gegebenenfalls durch Halogen ersetzt sind, oder

$R^1NHCO-$, wobei $R^1$ Wasserstoff oder eine $C_1$-$C_6$-Alkylgruppe ist, oder

$R^2CONH-$, wobei $R^2$ Wasserstoff oder eine $C_1$-$C_6$-Alkylgruppe ist, bedeutet;

X die Bedeutung

$$-(CH_2)_pNHCONH,$$

wobei p eine ganze Zahl von Null bis 2 ist,

$$-NHCONHCH_2, \quad -NHCOCH_2, \quad -NHCOO, \quad -CSNH,$$

$$-NHC(:NCN)NH$$

oder

$$-CH(A)CONH,$$

wobei A Halogen ist, hat;

Y $-(CH_2)_q-$, wobei q eine ganze Zahl von 1 bis 3 ist, oder -CH=CH- (E oder Z) darstellt;

Z eine $C_1$-$C_6$-Alkylgruppe gegebenenfalls substituiert durch eine oder mehrere unabhängig ausgewählte polare Gruppen bedeutet; und

Ring A gegebenenfalls durch ein oder mehrere Fluoratome substituiert ist;

mit der Maßgabe, daß

(i) in der Verbindung der Formel (I) $q \neq 1$ ist, wenn W unsubstituiertes Phenyl darstellt und n Null ist, und

(ii) die Verbindung der Formel (I) nicht 1-[2,4-Difluor-6-{2-[4-(2,2-dimethylpropyl)-phenyl]-äthyl}-phenyl]-3-heptylharnstoff ist, und Salze oder Solvate hievon.

2. Verbindung der Formel (I) nach Anspruch 1, worin

W gerades $C_7$-$C_{11}$-Alkyl, Cyclohexyl, Cyclohexylmethyl oder 1,1-Dimethylprop-2-inyl,

Phenyl, Benzyl,

$n$-$C_4H_9NHCO-$ oder

$n$-$C_3H_7CONH-$ bedeutet,

X die Bedeutung

$$-(CH_2)_pNHCONH,$$

wobei p eine ganze Zahl von Null bis 2 ist,

$$-NHCONHCH_2, \quad -NHCOCH_2, \quad -NHCOO, \quad -CSNH,$$

$$-NHC(:NCN)NH$$

oder

$$-CH(Br)CONH$$

hat;

Z Wasserstoff (d.h. m ist Null) oder, wenn m 1 ist, p-Isobutyl oder p-Neopentyl darstellt; und

Ring A unsubstituiert oder in 2,4- oder 3,5-Stellung durch Fluoratome disubstituiert ist,

und physiologische annehmbare Salze und Solvate hievon.

3. Verbindung der Formel (I) nach Anspruch 2, worin

W n-Heptyl bedeutet,

X die Bedeutung

$$-NHCONH, \quad -NHCOCH_2$$

oder

$$-NHCOO$$

hat;
Y $-(CH_2)_2-$ ist;
m 1 ist und Z p-Isobutyl oder p-Neopentyl darstellt; und
Ring A in der 2,4-Stellung durch Fluoratome disubstituiert ist,
und physiologisch annehmbare Salze und Solvate hievon.

4. Pharmazeutische Formulierung umfassend eine Verbindung der Formel (I), wie in Anspruch 1 beansprucht, oder ein physiologisch annehmbares Salz oder Solvat hievon, einen pharmazeutisch annehmebaren Träger oder Exzipienten und gegebenenfalls ein oder mehrere andere pharmakologisch aktive Mittel.

5. Formulierung nach Anspruch 4, in der die Verbindung der Formel (I) wie in Anspruch 2 beansprucht oder ein physiologisch annehmbares Salz oder Solvat hievon ist.

6. Formulierung nach Anspruch 4, in der die Verbindung der Formel (I) wie in Anspruch 3 beansprucht oder ein physiologisch annehmbares Salz oder Solvat hievon ist.

7. Formulierung nach einem der Ansprüche 4 bis 6, die in Form einer Tablette oder einer Kapsel ist.

8. Verfahren zur Herstellung einer Verbindung der Formel (I), wie in Anspruch 1 beansprucht, das das Umsetzen einer Verbindung der Formel (II)

W - P (II)

worin W wie in Anspruch 1 definiert ist, mit einer Verbindung der Formel (III)

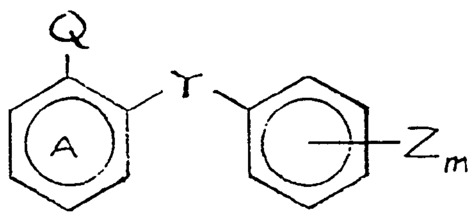

(III),

worin m, Y und Z wie in Anspruch 1 definiert sind und Ring A gegebenenfalls wie in Anspruch 1 definiert substituiert ist, entweder P in der Verbindung der Formel (II) oder Q in der Verbindung der Formel (III) eine nukleophile Gruppe ist, die zum Reagieren mit Q in der Verbindung der Formel (III) bzw. andererseits P in der Verbindung der Formel (II) imstande ist, um eine Verbindung der Formel (I) mit einer Bindung X, wie in Anspruch 1 definiert, zu ergeben,
und gegebenenfalls das Überführen der so gebildeten Verbindung der Formel (I) in ein entsprechendes Salz oder Solvat umfaßt.

9. Verbindung der Formel (I), wie in einem der Ansprüche 1 bis 3 beansprucht, oder ein physiologisch annehmbares Salz oder Solvat hievon zur Verwendung in der Therapie.

10. Verbindung der Formel (I), wie in einem der Ansprüche 1 bis 3 beansprucht, oder ein physiologisch annehmbares Salz oder Solvat hievon zur Verwendung bei der Prophylaxe oder Behandlung eines klinischen Zustandes, für den ein ACAT-Inhibitor angezeigt ist.

11. Verbindung der Formel (I), wie in einem der Ansprüche 1 bis 3 beansprucht, oder ein physiologisch annehmbares Salz oder Solvat hievon zur Verwendung bei der Prophylaxe oder Behandlung von Atherosklerose.

12. Verwendung einer Verbindung der Formel (I), wie in einem der Ansprüche 1 bis 3 beansprucht, oder eines physiologisch annehmbaren Salzes oder Solvats hievon bei der Herstellung eines Medikaments für die Prophylaxe oder Behandlung eines klinischen Zustandes, für den ein ACAT-Inhibitor angezeigt ist.

13. Verwendung einer Verbindung der Formel (I), wie in einem der Ansprüche 1 bis 3 beansprucht, oder eines physiologisch annehmbaren Salzes oder Solvats hievon bei der Herstellung eines Medikaments für die Prophylaxe oder Behandlung von Atherosklerose.

**Patentansprüche für folgende Vertragsstaaten : ES, GR**

1. Verfahren zur Herstellung einer Verbindung der Formel (I)

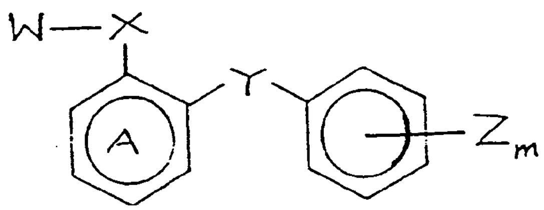

(I),

worin

m Null oder 1 ist,

W eine gerade, verzweigte oder cyclische $C_5$-$C_{16}$-Alkyl-, Alkenyl- oder Alkinylgruppe oder

$Ph(CH_2)_n$, wobei Ph Phenyl darstellt und n eine ganze Zahl von Null bis 2 ist, wobei die Phenylgruppe gegebenenfalls durch ein oder mehrere Atome oder Gruppen unabhängig ausgewählt aus Halogen, Hydroxy, Nitro, $C_1$-$C_4$-Alkoxy und $C_1$-$C_4$-Alkyl substituiert ist, wobei ein oder mehrere der Wasserstoffatome in der Alkylgruppe gegebenenfalls durch Halogen ersetzt sind, oder

$R^1NHCO$-, wobei $R^1$ Wasserstoff oder eine $C_1$-$C_6$-Alkylgruppe ist, oder

$R^2CONH$-, wobei $R^2$ Wasserstoff oder eine $C_1$-$C_6$-Alkylgruppe ist, bedeutet;

X die Bedeutung

$$-(CH_2)_pNHCONH,$$

wobei p eine ganze Zahl von Null bis 2 ist,

$$-NHCONHCH_2, \quad -NHCOCH_2, \quad -NHCOO, \quad -CSNH,$$

$$-NHC(:NCN)NH$$

oder

$$-CH(A)CONH,$$

wobei A Halogen ist, hat;

Y $-(CH_2)_q-$, wobei q eine ganze Zahl von 1 bis 3 ist, oder -CH=CH- (E oder Z) darstellt;

Z eine $C_1$-$C_6$-Alkylgruppe gegebenenfalls substituiert durch eine oder mehrere unabhängig ausgewählte polare Gruppen bedeutet; und

Ring A gegebenenfalls durch ein oder mehrere Fluoratome substituiert ist;

mit der Maßgabe, daß

(i) in der Verbindung der Formel (I) q ≠ 1 ist, wenn W unsubstituiertes Phenyl darstellt und n Null ist, und

(ii) die Verbindung der Formel (I) nicht 1-[2,4-Difluor-6-{2[4-(2,2-dimethylpropyl)-phenyl]-äthyl}-phenyl]-3-heptylharnstoff ist, welches Verfahren das Umsetzen einer Verbindung der Formel (II)

W - P (II),

worin W wie in Anspruch 1 definiert ist, mit einer Verbindung der Formel (III)

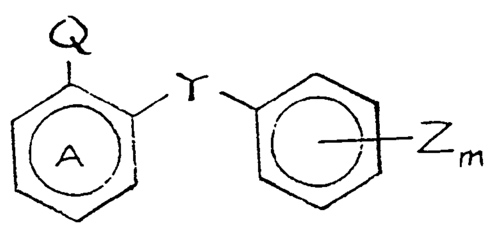

(III),

worin m, Y und Z wie in Anspruch 1 definiert sind und Ring A gegebenenfalls wie in Anspruch 1 definiert substituiert ist, entweder P in der Verbindung der Formel (II) oder Q in der Verbindung der Formel (III) eine nukleophile Gruppe ist, die zum Reagieren mit Q in der Verbindung der Formel (III) bzw. andererseits P in der Verbindung der Formel (II) imstande ist, um eine Verbindung der Formel (I) mit einer Bindung X, wie in Anspruch

1 definiert, zu ergeben,
und gegebenenfalls das Überführen der so gebildeten Verbindung der Formel (I) in ein entsprechendes Salz oder Solvat umfaßt.

2. Verfahren nach Anspruch 1 zur Herstellung einer Verbindung der Formel (I), wie in Anspruch 1 definiert, worin
W gerades $C_7$-$C_{11}$-Alkyl, Cyclohexyl, Cyclohexylmethyl oder 1,1-Dimethylprop-2-inyl,
Phenyl, Benzyl,
n-$C_4H_9$NHCO- oder
n-$C_3H_7$CONH- bedeutet,
X die Bedeutung

$$-(CH_2)_pNHCONH,$$

wobei p eine ganze Zahl von Null bis 2 ist,

$$-NHCONHCH_2,\ -NHCOCH_2,\ -NHCOO,\ -CSNH,$$

$$-NHC(:NCN)NH$$

oder

$$-CH(Br)CONH$$

hat;
Z Wasserstoff (d.h. m ist Null) oder, wenn m 1 ist, p-Isobutyl oder p-Neopentyl darstellt; und
Ring A unsubstituiert oder in 2,4- oder 3,5-Stellung durch Fluoratome disubstituiert ist,
und physiologisch annehmbarer Salze oder Solvate hievon.

3. Verfahren nach Anspruch 2 zur Herstellung einer Verbindung der Formel (I), wie in Anspruch 1 definiert, worin
W n-Heptyl bedeutet,
X die Bedeutung

$$-NHCONH,\ -NHCOCH_2$$

oder

$$-NHCOO$$

hat;
Y $-(CH_2)_2-$ ist;
m 1 ist und Z p-Isobutyl oder p-Neopentyl darstellt; und
Ring A in der 2,4-Stellung durch Fluoratome disubstituiert ist,
und physiologisch annehmbarer Salze oder Solvate hievon.

4. Verfahren nach Anspruch 1 zur Herstellung einer Verbindung der Formel (I), worin X die Bedeutung -NHCONH hat, in dem P in der Verbindung der Formel (II) eine Isocyanatgruppe und Q in der Verbindung der Formel (III) eine Aminogruppe ist.

5. Verfahren nach Anspruch 1 zur Herstellung einer Verbindung der Formel (I), worin X die Bedeutung -NHCONH$_2$ hat, in dem P in der Verbindung der Formel (II) eine Aminogruppe und Q in der Verbindung der Formel (III) eine Carboxymethylgruppe ist.

6. Verfahren nach Anspruch 1 zur Herstellung einer Verbindung der Formel (I), worin X die Bedeutung -NHCONH hat, in dem P in der Verbindung der Formel (II) eine Isocyanatgruppe und Q in der Verbindung der Formel (III) eine Hydroxygruppe ist.

7. Verfahren zum Herstellen einer pharmazeutischen Formulierung, das
(a) das Herstellen einer Verbindung der Formel (I) oder eines physiologisch annehmbaren Salzes oder Solvates hievon durch ein Verfahren nach einem der Ansprüche 1 bis 6 und
(b) das Mischen des Produktes von Schritt (a) mit einem pharmazeutisch annehmbaren Träger oder Exzipienten und gegebenenfalls einem oder mehreren anderen pharmakologisch aktiven Mitteln umfaßt.

8. Verfahren nach Anspruch 7, das den weiteren Schritt (c) umfaßt, in dem die Mischung von Schritt (b)

zu einer Tablette oder Kapsel geformt wird.

**Revendications**

**Revendications pour les Etats contractants suivants : AT, BE, CH, DE, FR, GB, IT, LI, LU, NL, SE**

1. Composé de formule (I) :

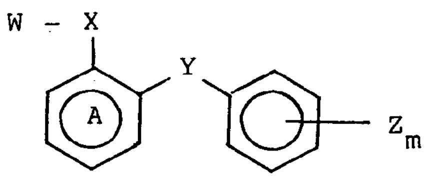

(I)

où
m est 0 ou 1;
W est un radical alcoyle, alcényle ou alcynyle cyclique ou en chaîne droite ou ramifiée en $C_{5-16}$, ou
Ph $(CH_2)_n$- où Ph est phényle et n est un entier de 0 à 2, le radical phényle étant facultativement substitué par un ou plusieurs atomes ou radicaux choisis indépendamment parmi halogène, hydroxyle, nitro, $C_{1-4}$-alcoxy et $C_{1-4}$-alcoyle, où un ou plusieurs des atomes d'hydrogène du radical alcoyle sont facultativement remplacés par halogène, ou
$R^1$NHCO-, où $R^1$ est hydrogène ou un radical $C_{1-6}$-alcoyle, ou
$R^2$CONH-, où $R^2$ est hydrogène ou un radical $C_{1-6}$-alcoyle;
X est $-(CH_2)_p$NHCONH-, où p est un entier de 0 à 2, $-NHCONHCH_2-$, $-NHCOCH_2-$, -NHCOO-, -CSNH-, -NHC(=NCN)NH- ou -CH(A)CONH- où A est halogène;
Y est $-(CH_2)_q-$, où q est un entier de 1 à 3, ou -CH=CH- (E ou Z) ;
Z est un radical $C_{1-6}$-alcoyle, facultativement substitué par un ou plusieurs radicaux polaires choisis indépendamment, et
le cycle A est facultativement substitué par un ou plusieurs atomes de fluor;
avec les restrictions :
(i) que dans le composé de formule (I), $q \neq 1$ lorsque W est Ph non substitué et n est 0, et
(ii) que le composé de formule (I) n'est pas la 1-[2,4-difluoro-6-{2-[4-(2,2-diméthylpropyl}phényl]éthyl]phényl]-3-heptylurée;
et ses sels ou solvates.

2. Composé de formule (I) suivant la revendication 1, dans lequel : W est $C_{7-11}$-alcoyle en chaîne droite, cyclohexyle, cyclohexylméthyle ou 1,1-diméthylprop-2-ynyle, ou
phényle ou benzyle, ou
n-$C_4H_9$NHCO-, ou
n-$C_3H_7$CONH-;
X est $-(CH_2)_p$NHCONH-, où p est un entier de 0 à 2, $-NHCONHCH_2-$, $-NHCOCH_2-$, -NHCOO-, -CSNH-, -NHC(=NCN)NH- ou -CH(Br)CONH-;
Z est hydrogène (c'est-à-dire m est 0) ou, lorsque m est 1, p-isobutyle ou p-néopentyle, et
le cycle A est non substitué ou disubstitué aux positions 2,4 ou 3,5 par des atomes de fluor;
et ses sels et solvates physiologiquement acceptables.

3. Composé de formule (I) suivant la revendication 2, dans lequel :
W est n-heptyle;
X est -NHCONH-, $-NHCOCH_2-$ ou -NHCOO-;
Y est $-(CH_2)_2-$;
m est 1 et Z est p-isobutyle ou p-néopentyle, et
le cycle A est disubstitué aux positions 2,4 par des atomes de fluor;
et ses sels ou solvates physiologiquement acceptables.

4. Composition pharmaceutique comprenant un composé de formule (I) suivant la revendication 1, ou un sel ou solvate physiologiquement acceptable de celui-ci, un excipient ou diluant pharmaceutiquement acceptable et facultativement un ou plusieurs autres agents pharmacologiquement actifs.

5. Composition suivant la revendication 4, dans laquelle le composé de formule (I) est un composé suivant la revendication 2 ou un sel ou solvate physiologiquement acceptable de celui-ci.

6. Composition suivant la revendication 4, dans laquelle le composé de formule (I) est un composé suivant la revendication 3 ou un sel ou solvate physiologiquement acceptable de celui-ci.

7. Composition suivant l'une quelconque des revendications 4 à 6, qui se trouve sous la forme d'un comprimé ou d'une capsule.

8. Procédé de préparation d'un composé de formule (I) suivant la revendication 1, qui comprend la réaction d'un composé de formule (II) :

$$W-P \quad (II)$$

où W est tel que défini dans la revendication 1, avec un composé de formule (III) :

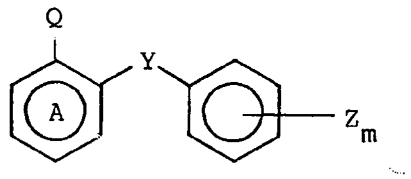

(III)

où m, Y et Z sont tels que définis dans la revendication 1 et le cycle A est facultativement substitué comme défini dans la revendication 1, P dans le composé de formule (II) ou Q dans le composé de formule (III) étant un radical nucléophile capable de réagir avec Q dans le composé de formule (III) ou, en variante, P dans le composé de formule (II), respectivement, pour donner un composé de formule (I) comprenant un élément de liaison X tel que défini dans la revendication 1;
et facultativement, la conversion du composé de formule (I) résultant en un sel ou solvate de celui-ci.

9. Composé de formule (I) suivant l'une quelconque des revendications 1 à 3, ou sel ou solvate physiologiquement acceptable de celui-ci, à utiliser en thérapeutique.

10. Composé de formule (I) suivant l'une quelconque des revendications 1 à 3, ou sel ou solvate physiologiquement acceptable de celui-ci, à utiliser dans la prophylaxie ou le traitement d'un état clinique pour lequel un inhibiteur de l'ACAT est indiqué.

11. Composé de formule (I) suivant l'une quelconque des revendications 1 à 3, ou un sel ou solvate physiologiquement acceptable de celui-ci, à utiliser dans la prophylaxie ou le traitement de l'athérosclérose.

12. Utilisation d'un composé de formule (I) suivant l'une quelconque des revendications 1 à 3, ou d'un sel ou solvate physiologiquement acceptable de celui-ci, dans la fabrication d'un médicament pour la prophylaxie ou le traitement d'un état clinique pour lequel un inhibiteur de l'ACAT est indiqué.

13. Utilisation d'un composé de formule (I) suivant l'une quelconque des revendications 1 à 3 ou d'un sel ou solvate physiologiquement acceptable de celui-ci dans la fabrication d'un médicament pour la prophylaxie ou le traitement de l'athérosclérose.

**Revendications pour les Etats contractants suivants : ES, GR**

1. Procédé pour préparer un composé de formule (I) :

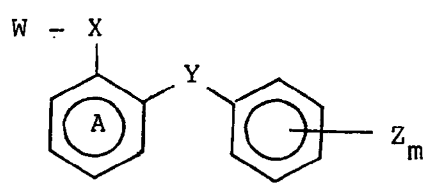

(I)

où
m est 0 ou 1;
W est un radical alcoyle, alcényle ou alcynyle cyclique ou en chaîne droite ou ramifiée en $C_{5-16}$, ou

$Ph(CH_2)_n$- où Ph est phényle et n est un entier de 0 à 2, le radical phényle étant facultativement substitué par un ou plusieurs atomes ou radicaux choisis indépendamment parmi halogène, hydroxyle, nitro, $C_{1-4}$-alcoxy et $C_{1-4}$-alcoyle, où un ou plusieurs des atomes d'hydrogène du radical alcoyle sont facultativement remplacés par halogène, ou

$R^1NHCO$-, où $R^1$ est hydrogène ou un radical $C_{1-6}$-alcoyle, ou

$R^2CONH$-, où $R^2$ est hydrogène ou un radical $C_{1-6}$-alcoyle;

X est $-(CH_2)_pNHCONH$-, où p est un entier de 0 à 2, $-NHCONHCH_2$-, $-NHCOCH_2$-, -NHCOO-, -CSNH-, -NHC(=NCN)NH- ou -CH(A)CONH- où A est halogène;

Y est $-(CH_2)_q$-, où q est un entier de 1 à 3, ou -CH=CH- (E ou Z);

Z est un radical $C_{1-6}$-alcoyle, facultativement substitué par un ou plusieurs radicaux polaires choisis indépendamment, et

le cycle A est facultativement substitué par un ou plusieurs atomes de fluor;

avec les restrictions :

(i) que dans le composé de formule (I), $q \neq 1$ lorsque W est Ph non substitué et n est 0, et

(ii) que le composé de formule (I) n'est pas la 1-[2,4-difluoro-6-{2-[4-(2,2-diméthylpropyl)phényl]éthyl}phényl]-3-heptylurée;

lequel procédé comprend la réaction d'un composé de formule (II) :

W - P (II)

où W est tel que défini ci-dessus, avec un composé de formule (III) :

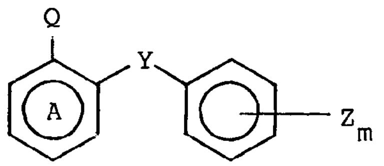

(III)

où m, Y et Z sont tels que définis ci-dessus et le cycle A est facultativement substitué comme défini ci-dessus, P dans le composé de formule (II) ou Q dans le composé de formule (III) étant un radical nucléophile capable de réagir avec Q dans le composé de formule (III) ou, en variante, P dans le composé de formule (II), respectivement, pour donner un composé de formule (I) comprenant un élément de liaison X tel que défini ci-dessus; et facultativement, la conversion du composé de formule (I) résultant en un sel ou solvate de celui-ci.

2. Procédé suivant la revendication 1, pour préparer un composé de formule (I) tel que défini dans la revendication 1, dans lequel :

W est $C_{7-11}$-alcoyle en chaîne droite, cyclohexyle, cyclohexylméthyle ou 1,1-diméthylprop-2-ynyle, ou

phényle ou benzyle, ou

n-$C_4H_9NHCO$-, où

n-$C_3H_7CONH$-;

X est $-(CH_2)_pNHCONH$-, où p est un entier de 0 à 2, $-NHCONHCH_2$-, $-NHCOCH_2$-, -NHCOO-, -CSNH-, -NHC(=NCN)NH- ou -CH(Br)CONH-;

Z est hydrogène (c'est-à-dire m est 0) ou, lorsque m est 1, p-isobutyle ou p-néopentyle, et

le cycle A est non substitué ou disubstitué aux positions 2,4 ou 3,5 par des atomes de fluor;

et ses sels et solvates physiologiquement acceptables.

3. Procédé suivant la revendication 2, pour préparer un composé de formule (I) tel que défini dans la revendication 1, dans lequel :

W est n-heptyle;

X est -NHCONH-, $-NHCOCH_2$- ou -NHCOO-;

Y est $-(CH_2)_2$-;

m est 1 et Z est p-isobutyle ou p-néopentyle, et

le cycle A est disubstitué aux positions 2,4 par des atomes de fluor;

et ses sels et solvates physiologiquement acceptables.

4. Procédé suivant la revendication 1 pour préparer un composé de formule (I), où X est -NHCONH-, dans lequel P dans le composé de formule (II) est un radical isocyanate et Q dans le composé de formule (III) est un radical amino.

5. Procédé suivant la revendication 1 pour préparer un composé de formule (I), où X est $-NHCOCH_2$-, dans lequel P dans le composé de formule (II) est un radical amino et Q dans le composé de formule (III) est un radical carboxyméthyle.

6. Procédé suivant la revendication 1 pour préparer un composé de formule (I), où X est -NHCOO-, dans lequel P dans le composé de formule (II) est un radical isocyanate et Q dans le composé de formule (III) est un radical hydroxyle.

7. Procédé pour préparer une composition pharmaceutique, qui comprend :

(a) la préparation d'un composé de formule (I) ou d'un sel ou solvate physiologiquement acceptable de

celui-ci par un procédé suivant l'une quelconque des revendications 1 à 6, et
(b) le mélange du produit du stade (a) avec un excipient ou diluant pharmaceutiquement acceptable et facultativement un ou plusieurs autres agents pharmacologiquement actifs.

8. Procédé suivant la revendication 7, qui comprend un stade supplémentaire (c) au cours duquel le mélange obtenu au stade (d) est façonné en un comprimé ou une capsule.